# EUROPEAN PATENT SPECIFICATION

(11) **EP 0 943 677 B1**
(45) Date of publication and mention of the grant of the patent: **09.11.2005**
(21) Application number: 99400362.2
(22) Date of filing: 16.02.1999
(51) Int. Cl.: C12M 1/26

(54) **Automated biogenerator**
Automatischer Biogenerator
Générateur biologique automatisé

(30) Priority: 27.02.1998 US 31642; 08.01.1999 US 227792
(43) Date of publication of application: 22.09.1999
(73) Proprietor: NCH Corporation, Irving, Texas 75062 (US)
(72) Inventor: Kiplinger, Dale Vilmer, Carrollton, TX 75007 (US); Pruitt, Judith Gayle, Mesquite, TX 75181 (US); Evaro, Jose Eduardo, Irving, TX 75062 (US); Pearce III, Robert Clarence, Arlington, TX 76001 (US)
(74) Representative: Pochart, François

(56) References cited:
- EP-A- 0 130 499
- DE-A- 19 507 456
- GB-A- 2 162 195
- US-A- 3 220 706
- US-A- 4 244 815
- US-A- 4 832 848
- US-A- 5 369 032

## Description

### TECHNICAL FIELD

The present invention relates to bacteria cultivation and dispensing systems, and more particularly to an automatic bacteria cultivation and dispensing system that is useful for incubating bacteria from a starter population to a utility population within a predetermined interval and thereafter dispensing sufficient bacteria to perform a desired utility. A preferred utility for the disclosed system is the removal of grease from grease traps in commercial food preparation establishments.

### BACKGROUND OF THE INVENTION

In the environment, bacteria are constantly working to naturally break down organic materials. This natural process generally causes some organic materials to eventually degrade into carbon dioxide and water. Under normal conditions, competition for resources, limited supplies of nutrients, and natural enemies can combine to inhibit rapid bacterial growth. By isolating selected strains of bacteria and providing a food source they prefer, bacteria can be made to multiply at a very fast rate. A large quantity of bacteria can be generated in this manner within a relatively short time. The bacteria can then be used in a wide variety of applications where the breakdown of organic materials is desirable.

One application where the breakdown of organic materials is particularly useful is in the maintenance of grease traps. Grease traps are required on virtually all commercial facilities that discard liquid or solid grease into a sewer system. Grease traps generally range from a capacity of about five gallons to over several thousand gallons. The majority of fast food kitchens are equipped with grease traps of about 1000 gallons. The system of drains used for grease traps is generally separate from the drains that carry away waste products from restrooms, spent drinking water, etc. Grease traps tend to collect not only oils and fats, but also various organic waste materials such as starches and vegetable waste products. Normally, a significant flow of wastewater is also introduced into the separate grease trap drainage system from kitchen drains where grease is often found. To prevent the wastewater from flushing grease into the city sewer system, grease traps are designed with a series of weirs that trap the grease within the containment vessel and allow wastewater to pass through the vessel on to a city treatment facility.

Inevitably, however, some of the grease in the grease trap passes into the city sewage system downstream from the restaurant. This does not create problems if the amount of grease passing into the sewer system is kept at a low level. Most city standards restrict the release of grease into sewer lines to approximately 250 ppm or less. If significant amounts of grease pass into the sewage system, the grease can cause blockages in the city pipes. When this occurs, the grease trap can overflow into the street, causing health problems.. City maintenance crews often have to dig up the pipes under the street to remove the blockage. The cost of this procedure is typically passed on to the restaurant that released the grease. The restaurant usually must also pay a fine. For repeat offenders, the blockage can result in closure of the facility.

To avoid such problems, the current practice is to periodically collect the solid grease that floats on the top of the grease trap. In addition, every four to eight weeks, a service company should remove grease and other solid material that has settled and accumulated in the bottom of the grease trap, and should steam strip the walls and weirs. The cost of this service varies depending on the geographical region and the contract agreed upon by the restaurant owner and the service company, but is substantially greater over time than will be required using the system and method disclosed herein. If the grease trap is not pumped out on a regular basis, the grease layer can form such a thick crust that it blocks the inlet line into the grease trap and causes wastewater to back up into the facility. Such a back-up can require closing the restaurant or facility until the problem is resolved. Because of the potential fines and the possibility of temporary or permanent closure, maintenance of grease traps is of great importance to the owners of commercial food preparations establishments.

There are currently several products on the market that purportedly reduce the number of pump-outs needed. Many of these products are solvent-based or are detergents containing enzymes that will allegedly make the grease trap maintenance free. While many of these solvents or detergent products will dissolve the grease in the grease trap, the liquified grease often resolidifies a few feet down the sewage pipes, thereby blocking the flow of wastewater.

Another known device for treating grease traps uses bacteria in an attempt to digest the grease. The device includes a five gallon bucket that contains a bacterial gel material. Water continuously flows through the bucket and into the drain system. A disadvantage of this device is that most of the bacteria is introduced into the grease trap during periods of high kitchen activity. The volume of wastewater that flows through the grease trap flushes most of the bacteria through the grease trap and into the sewer system before the bacteria is able to digest the grease. In addition, a typical grease trap is generally a poor environment for growing bacteria rapidly due to a lack of oxygen, as well as the presence of contaminants such as detergents and antibacterial chemicals used in cleaning operations.

Another known treatment is to introduce preserved bacteria into the grease trap. This type of bacteria is generally in the form of a dry powder that consists of dormant bacteria spores. Before the growth of bacterial colonies can occur, these dormant spores must go through an incubation period to form active vegetative cells. This process takes about six hours to occur. If the spores are introduced into the grease trap before this time, most of the bacteria will be flushed from the grease trap before digestion can occur.

Another known method of maintaining a grease trap is to grow large quantities of active bacteria offsite using a filtered air supply, distilled water, and a specially designed growth chamber. The large amount of bacteria needed to sufficiently digest the grease in the grease trap, however, has not been affordable because large volumes of bacteria are expensive to produce and difficult to transport.

Prior art devices said to be useful for aerating a cultivation medium within a tank, vessel or container to promote fermentation or bacterial growth are disclosed, for example, in United States Patent Nos. 4,051,204; 4,426,450; 4,883,759; and 4,888,294.

U.S. 5,369,032 discloses an apparatus for preparing at a livestock feedlot a concentrated suspension of anaerobic bacteria at a known, accurate concentration and for storing the prepared suspension for prolonged periods under ready-to-use conditions without significant loss of viability. A mixer offset from the center is operated at high speed during addition of bacteria to facilitate dispersion and operates periodically thereafter.

U.S. 4,244,815 discloses a process and apparatus for the purification of effluent waters containing organic pollutants by a rapid biological oxidation process characterized in that the liquid to be purified is introduced into a tank containing a liquid having a high biomass concentration, which is then recirculated until the impurities are removed as sludge.

DE 195 07 456 A1 discloses an apparatus and method for the continuous culture of aerobic organisms at high cell density in a vortex reactor into which a liquid medium is injected tangentially in the upper part of the reactor. Aeration is provided by an eductor disposed upstream of the tangential inlet and/or by subsurface, perforated gas distributors.

EP 130499 discloses an appliance for the biological treatment of sewage inside a vertical tank with an overflow on the outer shell which defines the space for the liquid and gas. The sewage is injected through a nozzle below liquid level into a guide cylinder inside which it descends and circulates in a loop.

U.S. 4,832,848 discloses a tower designed to improve the dispersion of air or oxygen in liquid sewage or sludge. The tower includes a plurality of vertically stacked annular chambers disposed around an upright return conduit. Sewage is pumped into the lowermost chamber in combination with pressurized air and spirals upwardly until it reaches the first horizontal barrier. The dispersed mixture then flows upwardly into the next higher chamber through passages and is discharged tangentially by throttling elbow members. Upon reaching the top chamber, most of the liquid passes through nipples into a central vertical conduit through which it flows downwardly to the intakes of recirculation pumps. Because the gaseous fraction tends to separate and collect in the top chamber, much of it is channeled into an overhead receptacle where the pressure is reduced to atmospheric and much of the gas is vented.

U.S. 3,220,706 discloses a sewage treatment system and more particularly an aeration tank for use in such a system. Sewage is introduced into an aeration tank through tangential inlet nozzles and pressurized air is supplied to the circulating sewage through header ring and upwardly directed nozzles. The combined effects of the upward air velocity and the circular movement of the tank's liquid caused by the tangential entry of the liquid into the tank produces an upward spiraling motion of the liquid and air.

GB 2 162 195 A discloses an apparatus and method for anaerobic fermentation of organic waste material capable of being biodegraded into useful gaseous products. The apparatus consists of a multiple tank arrangement having a flexible membrane or cover securely attached to the top to collect gaseous products. The multiple tank arrangement defines a tortuous path for the organic waste material to allow sufficient time for the fermentation to take place.

### SUMMARY OF THE INVENTION

The present invention comprises an automated biogeneration system for producing and dispensing liquid concentrates of active bacteria at predetermined intervals. According to a preferred embodiment of the invention, bacteria produced in this manner can be used to digest organic material in a grease trap and to reduce the frequency of pump-outs required. The system and method of the invention can also be used to supply bacteria for many other useful applications as disclosed below.

One may describe a method for growing and selectively discharging bacteria is disclosed whereby water and a predetermined quantity of a powdered mixture of dehydrated "starter" bacteria and appropriate nutrient(s) are automatically introduced into a biogeneration chamber for the purpose of growing and quickly multiplying the selected bacteria. Multiple strains of bacteria can be used as long as the nutrient package is designed to support each of the multiple strains. Pressurized air is supplied to the chamber to support aerobic bacterial reproduction, and is desirably introduced according to a special method using a vortex that controls foaming within the biogeneration chamber. After the mixture is placed in the biogeneration chamber, the bacteria are permitted to grow and reproduce for a desired time, such as about 24 hours, while continually withdrawing liquid from the bottom of the chamber, recirculating it with a pump, and reintroducing it into the chamber in a tangentially directed flow to create the desired vortex. At the end of the growing period, the active bacteria are preferably discharged from the biogeneration chamber to another holding vessel or, more preferably, directly to a use site such as a restaurant grease trap. Once the contents of the biogeneration chamber are discharged, the process is repeated. The cycle of operation is desirably controlled by an electronic timer having relays that activate and deactivate switches and valves in accordance with predetermined parameters. Significant increases in bacterial production are observed using the system and method disclosed herein as compared to applicants' previously disclosed system and method.

According to another preferred embodiment of the invention, an automated batch system for growing and selectively discharging a bacteria-containing fluid is disclosed that comprises a biogeneration chamber having a substantially cylindrical sidewall, a top and a conical bottom, a feed source communicating with a feed inlet port in the top, a water source communicating with a water inlet port in the top, a pressurized air source communicating with an air inlet port in the top, a vent line communicating with a vent port in the top, a centrally disposed outlet port in the conical bottom, an orifice element disposed in the conical bottom at or near the outlet port, a recirculated fluid inlet port positioned and directed so as to reintroduce recirculated fluid into the chamber in a substantially tangential direction relative to the inside wall, a recirculating pump, flow tubing placing the recirculating pump inlet in fluid communication with the chamber outlet port and placing the recirculating pump outlet in fluid communication with the recirculated fluid inlet port, and a valve disposed in the flow tubing between the recirculating pump and the recirculated fluid inlet port to selectively divert flow from the pump to a drain line also communicating through the pump and the valve with the chamber outlet port.

### BRIEF DESCRIPTION OF THE DRAWINGS

The preferred embodiments of the invention are further described with reference to the accompanying drawings wherein:
FIG. 1 is a simplified schematic view of a biogeneration system made in accordance with the present invention;
FIG. 2 is an enlarged, simplified front elevation view of the biogeneration chamber shown in FIG. 1; and
FIG. 3 is an enlarged, simplified front perspective view, partially broken away, depicting one orifice element as installed in the bottom of a biogeneration chamber for use in the apparatus and method of the invention.

### DESCRIPTION OF THE PREFERRED EMBODIMENT

The automated biogeneration system and method of the present invention are useful for rapidly growing a relatively large supply of selected strains of bacteria using a relatively small quantity of starter bacteria. The method of the invention provides a low maintenance, hands-free process for growing bacteria that, depending upon the bacterial strains selected, are useful in a variety of applications. Although other possible applications for the system and method disclosed herein are discussed below, a particularly preferred application is the cultivation of bacteria for use in digesting grease in grease traps such as those used in the restaurant industry. If a large enough supply of suitable, active bacteria can be introduced into a grease trap on a frequent basis, the grease accumulation inside the trap can be controlled at levels that permit less frequent pump-outs and steam cleaning of the type now regularly required. This reduces service fees and facilitates compliance with regulations governing the amount of grease that can be released into municipal sewer systems.

In accordance with a preferred embodiment of the invention, an automatic system and method are disclosed for creating large batches of active bacteria that thrive on grease and other organic matter, such as starches, sugars, and proteins. The automated function reduces labor costs, and the biogeneration chamber provides a favorable environment for growing bacteria. When introduced into a grease trap, generally through a drain line downstream of the P-trap, the large volume of bacteria produced digests some of the organic materials in the grease trap, producing carbon dioxide and water as the principal waste products.

The starter bacteria and nutrients needed to support growth and reproduction of the bacteria within the biogeneration chamber are easily transported, most preferably in powder form, making the transport of large supplies of bacteria unnecessary. The subject method does not require precise control of the temperature; nor is it necessary to use a filtered air supply or distilled water. Because multiple strains of desirable bacteria can be grown simultaneously in the same biogeneration chamber, they are capable of digesting a variety of organic materials found in grease traps while simultaneously reducing odor problems. Because they are grown onsite, the useful bacteria are active upon introduction into a grease trap. Furthermore, by introducing the bacteria during periods of low kitchen activity, the bacteria's residence time in the grease trap can be maximized.

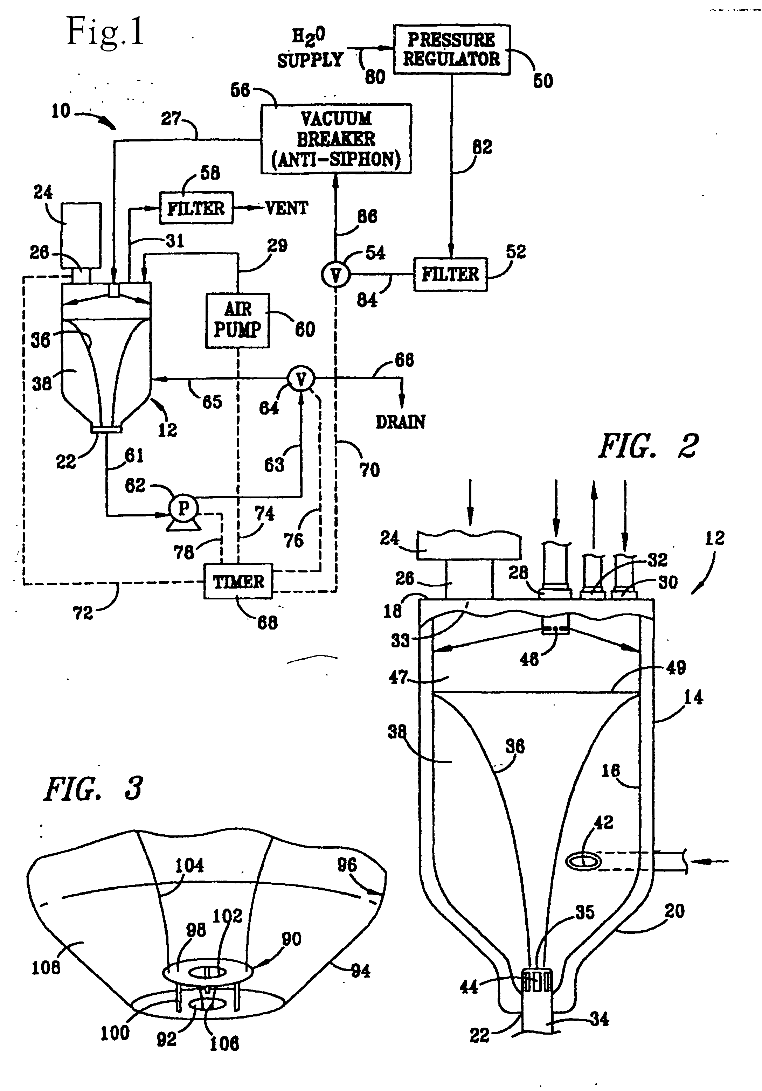

Referring to FIG. 1, system 10 of the invention preferably comprises biogeneration chamber 12, a feed source 24 of starter bacteria and nutrients, a feed control device 26, a water supply, a pressurized air supply, fluid recirculation pump 62, solenoid valves 54 and 64, timer 68, flow conduits 27, 29, 31, 61, 63, 65, 66, 82, 84 and 86, and control lines 70, 72, 74, 76 and 78 respectively linking timer 68 to valves 54 and 64, air pump 60, feed control device 26 and fluid recirculation pump 62. The water supply is preferably fresh potable water provided to biogeneration chamber 12 at ambient temperature through flow line 80, pressure regulator 50, flow line 82, filter 52, flow line 84, water inlet solenoid valve 54, anti-siphon vacuum breaker 56 and flow line 27. The pressurized air supply is preferably an air pump 60, such as an aquarium air pump, capable of delivering to biogeneration chamber 12 a sufficient supply of air to support rapid bacterial growth at a pressure sufficient to enter the biogeneration chamber without disrupting the vortex 36 created therein as discussed below. The required air flow will of course vary according to the volume of biogeneration chamber 12 and the volume of the bacteria-containing fluid mixture 38 within the chamber. Vent line 31 is provided to prevent pressure build-up inside biogeneration chamber 12 and is preferably filtered by filter 58, and exhaust to atmosphere or some other type of recovery unit as desired or that may be required under special circumstances. Vent line 31 can be connected to an existing drain system downstream from the P-trap to allow venting of biogeneration chamber 12 without releasing bacteria into the kitchen area. If connected to a drain, vent line 31 can also comprise a conventional diaphragm check valve or backflow preventer (not shown in FIG. 1) to prevent unwanted bacteria from entering the biogeneration chamber 12 from the drain.

Feed source 24 is desirably a hopper or other container capable of maintaining and controllably releasing through feed control device 26 a mixture of starter bacteria useful for the intended application and nutrients needed for the starter bacteria to grow and replicate rapidly within biogeneration chamber 12. According to a particularly preferred embodiment of the invention, the starter bacteria and nutrients are provided together in a premixed powder that is activated when hydrated and mixed in the presence of air. While the use of a dry, premixed powder feed is preferred, liquid feeds can also be used in the subject system and method, and the nutrients do not necessarily have to be premixed with the starter bacteria. Feed control device 26 can be any device or combination of devices suitable for introducing starter bacteria and nutrients into biogeneration system 12 in a controlled manner and can include, for example, a rotary valve, sliding volumetric gate, vibrating roller mechanism or weigh-belt conveyor. Alternatively, premixed powder can also be aspirated into the water inlet line using a venturi arrangement. Although a single feed source 24 and feed control device 26 are depicted in FIG. 1, it is understood that a plurality of such feed sources and/or feed control devices can also be used within the scope of the invention.

Fluid recirculation pump 62 withdraws fluid mixture 38 from biogeneration chamber 12 through outlet port 22 and line 61, and depending upon the setting of three-way solenoid valve 64, either returns the fluid mixture to biogeneration chamber 12 through lines 63 and 65 or else discharges the fluid mixture to a drain or other receptacle through lines 63 and 66. Fluid recirculation pump 62 is desirably a centrifugal pump, although it is understood that diaphragm pumps and other similarly effective fluid recirculation devices can also be used within the scope of the invention. Diaphragm pumps are self-priming and are less affected by excess air in the fluid mixture. Fluid recirculation pump 62 is depicted in FIG. 1 as being controlled by timer 68, but it is understood that pump 62 can also be controlled, for example, by electrical, mechanical, optical or ultrasonic level indicators or sensors in biogeneration chamber 12 and related switches as needed.

Timer 68 is desirably a conventional electronic control device capable of timing multiple events in the cycle of operation and signaling switches in feed control device 26, air pump 60, solenoid valves 54 and 64, and fluid recirculation pump 62 in accordance with the method of the invention as described below. It will appreciated upon reading this disclosure, however, that some or all of the control functions performed by timer 68 can also be performed within the scope of the invention by the use of multiple timers, a program logic controller, a custom designed programmable circuit board, a PC based controller, pneumatic controllers, level controllers and other similarly effective means. Timer 68 will desirably accept and react to inputs from conductivity sensors, mechanical floats, optical sensors and ultrasonic sensors, and may also be capable of simultaneously operating other equipment or apparatus, whether or not depicted in FIG. 1 or described herein.

A preferred biogeneration chamber 12 for use in system 10 and the method of the invention is further described and explained in relation to FIG. 2. Biogeneration chamber 12 preferably comprises a substantially cylindrical sidewall 14 with interior surface 16 that is smoothly and continuously joined to a conical lower section 20 having a centrally disposed chamber outlet port 22 at its lower end. Top 18 may be flat, domed or otherwise shaped, and is desirably removable to permit periodic cleaning of inside surface 16 of biogeneration chamber 12. Water inlet port 28, air inlet port 30, feed inlet port 33 and vent port 32 are desirable provided in top 18 to communicate with water inlet line 27, air inlet line 29, feed control device 26 and vent line 31, respectively. Disperser nozzle 46 is preferably provided in water inlet port 28 to direct water introduced into biogeneration chamber 12 in a 360° spray against interior surface 16, and can also function as a rinse nozzle during clean-out of chamber 12 if desired. Orifice element 34 is preferably provided in chamber outlet port 22 to assist in controlling the amount of air entering line 61 (FIG. 1) from vortex 36 in fluid medium or mixture 38. Too great a restriction in orifice element 34 can starve fluid recirculation pump 62 and promote plugging of the orifices.

Applicants have discovered that vortex 36 within biogeneration chamber 12 is an effective tool for mixing the bacteria and nutrients in the fluid medium and for aerating fluid mixture 38 without causing the significant amount of undesirable foam that is produced in prior art systems when air is bubbled into a biogenerator beneath the surface of a fluid mixture. The amount of aeration achieved with vortex 36 also significantly surpasses the aeration achieved when air is introduced above the fluid surface without a vortex. Vortex 36 is preferably created by the continuous reintroduction into biogeneration chamber 12 of a portion of fluid mixture 38 that is withdrawn through outlet port 22 and recirculated by pump 62 through line 63, valve 64 and line 65 to recirculated fluid outlet port 42 disposed closely adjacent to interior surface 16, most preferably above conical section 20. Port 42 preferably communicates with the interior of biogeneration chamber 12 in a direction and in such manner that a continuous stream of recirculated fluid mixture 38 is reintroduced into biogeneration chamber 12 and directed horizontally along interior surface 16 in a direction that is characterized herein as being "substantially tangential" to interior surface 16. Although the term "tangential" ordinarily refers to a line tangent to a point on the circumference of a circle or cylinder that is directed away from the curve, the term is used herein to describe a curved flowpath, initially established in a substantially horizontal direction around inside surface 16 of biogeneration chamber 12, that diverges from horizontal as it continues around interior surface 16 and creates a downwardly spiraling vortex 36 in the center of biogeneration chamber 12. Port 42 can be disposed in a nozzle built into sidewall 14 of biogeneration chamber 12 or can be disposed at the end of a line extending interiorly past interior surface 16 as shown in FIG. 2. Although port 42 is shown as being elliptical in shape in FIG. 2, it will be understood that other shapes can also be used within the scope of the invention.

Orifice element 34 as shown in FIG. 2 is a cylindrical body having a top with a smaller-diameter orifice 35 and a plurality of rectangular, slot-like orifices 44 circumferentially spaced around its perimeter. It is to be understood that other orifice configurations can also be used within the scope of the present invention. As discussed below in relation to the method of the invention, orifice element 34 is useful for permitting fluid mixture 38 to be drawn into and through chamber outlet port 22 without permitting vortex 36 to continue downwardly into and through outlet port 22, which could promote cavitation on the inlet side of fluid recirculation pump 62.

FIG. 3 discloses an alternative structure for an orifice element 90 disposed adjacent to chamber outlet port 92 inside conical section 94 of an otherwise similar biogeneration chamber 96. Orifice element 90 comprises a horizontally disposed orifice plate 98 supported by legs 100 and vertically separated at a slight distance above the chamber outlet port 92.Orifice plate 98 desirably has an outside diameter greater than the diameter of the chamber outlet port 92 and comprises a single centrally disposed aperture 102 having a diameter smaller than the diameter of the chamber outlet port 92. Orifice plate 98 supports vortex 104 slightly above outlet port 92, permitting fluid mixture 108 to be drawn into outlet port 92 around and beneath disc 98, where a smaller diameter vortex 106 is created that is less likely to cause surging, or cavitation of a fluid recirculation pump.

While the components of the invention will understandably be sized, configured and constructed according to the intended manner and place of use, satisfactory results are achieved using a one gallon bioreactor chamber 12 with a conical bottom as discussed below, a fill level within the chamber corresponding to a fluid volume ranging from about 1.5 to about 3 liters, a fluid recirculation pump 62 rated at about 3 gallons per minute, and a 50 micron inlet water filter 52. According to a particularly preferred embodiment of system 10 of the invention for use in a typical restaurant environment to generate bacteria for discharge into a grease trap, the system components as described above can be mounted inside a cabinet and locked to prohibit access except by authorized personnel. During normal operation, infrequent access to the system is needed in order to periodically replenish the starter bacteria and nutrients. Although the time for replenishing starter bacteria and nutrients will vary according to the volume of feed source 24 and the volume of biogeneration chamber 12, monthly replenishment should be achievable in most instances. Infrequent removal and cleaning of biogeneration chamber 12 may also be desirable following a specified number of cycles of operation, which is also anticipated to be no more often than about once a month.

According to a preferred method of the invention, described herein in relation to FIGS. 1 and 2, a dry powder containing selected bacteria cultures and dry nutrients such as sugar, refined milk protein, corn starch, and bran is loaded into feed source 24. Desirable starter bacteria for controlling grease in a grease trap preferably include bacillus, pseudomonas, enterobacter and mixtures thereof. These organisms are known to digest various types of organic waste products that are commonly found in grease traps. Preferably, the starter bacteria is stabilized with a preservative and is inactive until it is diluted with water.

At the system start-up, timer 68 activates water solenoid valve 54, causing water to enter biogeneration chamber 12 through inlet port 28. After passing through vacuum breaker 56, which prevents fluid from chamber 12 from being siphoned back into the potable water system, water is sprayed into the biogeneration chamber, preferably a polyethylene container, through nozzle 46. Water flow is continued for a predetermined time or until fluid level 49 reaches a desired point, at which time valve 54 is closed to stop the flow. Timer 68 activates feed control device 26 to dispense a predetermined quantity of powder containing starter bacteria and nutrients into biogeneration chamber 12 through suitably sized inlet port 33, thereby hydrating and activating the starter bacteria. Air pump 60 is also activated by timer 68 and the slight positive pressure inside biogeneration chamber 12 effectively prevents unwanted bacteria from entering the biogeneration chamber after the initial start-up. Air pump 60 pumps fresh air into headspace 47 above liquid level 49, making air readily available for vortex 36 to siphon into fluid recirculating pump 62. The introduction of air into headspace 47 also helps carry off any gases created by the bacteria out through vent line 31. Although some unwanted bacteria may be introduced through the air supply or water supply, growth of unwanted organisms in relatively small numbers is not usually seriously detrimental to the process and can sometimes be suppressed by carefully selecting nutrients preferred by the desired bacteria.

Once the biogeneration chamber 102 is charged with water, starter bacteria, nutrients and air, fluid recirculation pump 62 is activated, and cultivation of the selected bacteria begins. Fluid recirculation pump 62 continuously draws a stream of fluid mixture 38 from conical bottom 20 of biogeneration chamber 12, through orifice element 34 in outlet port 22 and line 61, to the inlet side of the pump. Fluid circulation pump 62 discharges the pressurized fluid mixture 38 into line 63, and through three-way valve 64, which is automatically set to return fluid mixture 38 through line 65 to inlet port 42. As fluid mixture 38 is expelled from inlet port 42, fluid mixture 38 is cause to swirl inside chamber 12, desirably creating vortex 36 when the recirculation rate is properly adjusted, as for example, by controlling the pumping rate of pump 62.

When properly adjusted in order to promote mixing and aeration, the bottom of vortex 36 will desirably extend downward to orifice element 34 in outlet port 22. Orifice element 34 preferably prevents too much air or too little fluid mixture from entering line 61. Too much air can damage the rotor of a centrifugal pump and, if excessive, stall the pump. Some cavitation at pump 62 may be desirable for mixing and aeration, and partial cavitation at the pump inlet can beat air into the liquid, producing a thick froth. So long as it does not overfill biogenerator chamber 12, some amount of foam can be desirable, increasing aeration, and also increasing the available surface area. With the present system and method, any excess foam that is created is drawn back into vortex 36 within chamber 12 and reinjected into fluid mixture 38. The ratio of air to liquid entering line 61 can be adjusted by modifying the orifice element 34 or the recirculation rate through pump 62. Through use of the present system and method, vortex 36 in conjunction with fluid recirculation pump 62 provides excellent mixing, aeration and foam control.

After bacterial cultivation, i.e. recirculation, has continued for a desired period, for example about one day, timer 68 or another similarly effective means causes the flow of pressurized air into the chamber to be terminated and three-way valve 64 to redirect the flow of fluid mixture 38 discharged by fluid recirculation pump 62 into drain line 66, which can be directed to a use site or to an intermediate storage vessel. Once fluid level 49 has been pumped down to a desired point, pump 62 is deactivated and valve 64 is returned to its former position. Valve 54 is then reopened and the cycle of operation is repeated.

Although application of the invention to treat grease in a grease trap is one preferred embodiment, the invention can be equally useful in a number of other applications where large quantities of bacteria are needed. The following list includes several other illustrative examples where the invention can be used:
(1) Growing bacteria for breaking down manure and urine in cattle barns and feed lots. Use of bacteria in this manner results in a digested material that can be put directly on fields for use as a fertilizer. The pre-digestion of manure and urine allows the fertilizers and nutrients to be released into the soil much faster and with less odor problems than handling the waste material in raw form.
(2) Bacterial decomposition of agricultural waste products such as sugar cane stalks and corn stalks that are slow to degrade when left in the fields.
(3) The biological treatment of oil spills. Such treatment has generally been very expensive because of the high cost of producing live bacteria. The present invention, if used in a larger, scaled-up version, can provide a cost-effective method of growing the necessary live bacteria onsite in quantities sufficient to quickly reduce the damage to the environment.
(4) Converting PCB's created by waste materials from transformer cooling oils into less harmful substances that can be further treated. This conversion reduces the environmental problems associated with current disposal practices. Treatment of these hazardous materials is currently very expensive.
(5) Use of bacteria to accelerate the decomposition of human waste as found in portable rest rooms and in septic systems.
(6) Use of bacteria to feed on algae that forms on cooling towers and in ponds and fountains. This use would reduce the need for the use of heavily regulated toxic chemicals.
(7) Using live bacteria to control insects that infest fruit and vegetable crops, aiding in insect and disease control.
(8) Using live bacteria to control fungi in turf for golf courses, on lawns, and in other plant life. In this application, the liquid bacteria mixture could be applied directly into the irrigation system. The addition of fertilizers to the mixture would also enhance the growth of the bacteria.
(9) Treatment of soil after underground oil or gas spills have occurred. The live bacteria or other organisms could be used to more efficiently treat an oil or chemical spill area than relying on the natural bacteria in the soil to degrade the spill.
(10) Production of active yeast products for commercial bakeries.
(11) Production of yeasts used in the fermentation and production of alcohol-related products.
(12) Decomposition of industrial organic waste products before being discharged down the drains. Food and bottling plants are often assessed large fines for discharging large amounts of fats, oils, starches, and sugar-based products in excess of permissible discharge levels. The system of the present invention can be used to digest such waste products.

While the system, apparatus and method of the invention are disclosed herein in relation to their preferred embodiments, other alterations and modifications of the invention will become apparent to those of ordinary skill in the art upon reading this disclosure, and it is intended that the invention disclosed herein be limited only by the broadest interpretation of the appended claims to which the inventors are legally entitled. Those skilled in the art will also recognize upon reading this disclosure that the physical size, placement and types of bacteria, nutrient, water and air supply devices, control devices, containers and pumps can be varied or modified within the scope of the invention to meet the needs of a particular application.

## Claims

1. An automated system (10) useful for growing bacteria from a starter population to a utility population within a predetermined interval and thereafter dispensing the bacteria to perform a desired utility, the system comprising :
a biogeneration chamber (12) having a cylindrical sidewall with an inside surface, a top and a conical bottom ; the top further comprising feed, water and air inlet ports and a vent port ; a centrally disposed chamber outlet port (22,92) in the conical bottom; an orifice element (34,90) disposed inside the conical bottom near the outlet port ; and a recirculated fluid inlet port (42) in the sidewall at a point above the conical bottom, the recirculated fluid inlet port (42) being directed tangentially along the inside surface of the sidewall;
at least one timer (68);
at least one bacteria and nutrient feed source (24) communicating with the feed inlet port, said source being controllable by said timer;
a water source (80, 50, 82) communicating with the water inlet port through a valve that is controllable by said timer;
a pressurized air supply (60) communicating with the air inlet port, the pressurized air supply being controllable by said timer;
a vent line (31) communicating with the vent port;
a recirculating pump (62) having an inlet communicating with the chamber outlet port (22) and an outlet communicating with the recirculated fluid inlet port (42); and
a valve (64) disposed between the recirculating pump and the recirculated fluid inlet port (42) that is controllable by said timer to divert flow from the recirculating pump to a drain line that also communicates selectively with the chamber outlet port (22,92) through the valve and the recirculating pump.

2. The automated system of claim 1, wherein the bacteria and nutrient feed source communicates with the feed inlet through a dispensing apparatus.

3. The automated system of claim 1 or 2, wherein the dispensing apparatus is a valve controlled by the timer.

4. The automated system of claim 1 or 2, wherein the dispensing apparatus is a conveyor controlled by the timer.

5. The automated system of any one of claims 1 to 4, comprising independently controllable bacteria and nutrient feed sources.

6. The automated system of any one of claims 1 to 5, wherein the water supply is filtered and pressure regulated.

7. The automated system of any one of claims 1 to 6, wherein the pressurized air supply is an aquarium air pump.

8. The automated system of any one of claims 1 to 7, wherein the orifice element (90) comprises a horizontally disposed orifice plate (98) vertically separated at a slight distance above the chamber outlet port (92).

9. The automated system of claim 8, wherein the orifice plate (98) has an outside diameter greater than the diameter of the chamber outlet port (92) and comprises a single aperture (102) having a diameter smaller than the diameter of the chamber outlet port (92).

10. The automated system of any one of claims 1 to 7, wherein the orifice element is a cylindrical structure having a diameter approximatively equal to the diameter of the chamber outlet port and comprises a plurality of orifices disposed on its periphery that provide fluid communication between the interior of the chamber and the chamber outlet port.

11. The automated system of any one of claims 1 to 10, wherein the recirculating pump is a centrifugal pump.

12. The automated system of any one of claims 1 to 10, wherein the recirculating pump is a diaphragm pump.

## Patentansprüche

1. Automatisiertes System (10), das brauchbar ist zum Züchten von Bakterien aus einer Starterpopulation zu einer Nutzpopulation innerhalb eines vorbestimmten Zeitraums und zum danach Verteilen der Bakterien, um einen erwünschten Nutzen zu erfüllen, wobei das System aufweist:
einen Bioentwicklungsraum (12), der eine zylindrische Seitenwand mit einer Innenoberfläche, eine Oberseite und eine konische Unterseite; wobei die Oberseite außerdem Einspeise-, Wasser- und Lufteinlaß-Öffnungen und eine Entlüftungsöffnung aufweist; eine in der konischen Unterseite mittig angeordnete Raum-Auslaßöffnung (22, 92); ein innerhalb der konischen Unterseite nahe der Auslaßöffnung angeordnetes Öffnungselement (34, 90); und eine Einlaßöffnung (42) für rückgeführtes Fluid in der Seitenwand an einem Punkt oberhalb der konischen Unterseite, wobei die Einlaßöffnung (42) für rückgeführtes Fluid tangential entlang der Innenoberfläche der Seitenwand ausgerichtet ist; hat;
mindestens einen Zeitprogrammgeber (68);
mindestens eine Einspeisequelle (24) für Bakterien und Nährstoffe, die mit der Einspeise-Einlaßöffnung kommuniziert, wobei die Quelle durch den Zeitprogrammgeber steuerbar ist;
eine Wasserquelle (80, 50, 82), die mit der Wasser-Einlaßöffnung über ein Ventil, das durch den Zeitprogrammgeber steuerbar ist, kommuniziert;
eine druckbeaufschlagte Luftzuführung (60), die mit der Luft-Einlaßöffnung kommuniziert, wobei die druckbeaufschlagte Luftzuführung durch den Zeitprogrammgeber steuerbar ist;
eine Entlüftungsleitung (31), die mit der Entlüftungsöffnung kommuniziert;
eine Rückführpumpe (62) mit einem Einlaß, der mit der Raum-Auslaßöffnung (22) kommuniziert, und einem Auslaß, der mit der Einlaßöffnung (42) für rückgeführtes Fluid kommuniziert; und
ein zwischen der Rückführpumpe und der Einlaßöffnung (42) für rückgeführtes Fluid angeordnetes Ventil (64), das von dem Zeitprogrammgeber steuerbar ist, um Strömung von der Rückführpumpe zu einer Ablaufleitung, die ebenfalls über das Ventil und die Rückführpumpe selektiv mit der Raum-Auslaßöffnung (22, 92) kommuniziert, umzuleiten.

2. Automatisiertes System nach Anspruch 1, bei dem die Einspeisequelle für Bakterien und Nährstoffe über eine Verteilungsvorrichtung mit dem Einspeise-Einlaß kommuniziert.

3. Automatisiertes System nach Anspruch 1 oder 2, bei dem die Verteilungsvorrichtung ein von dem Zeitprogrammgeber gesteuertes Ventil ist.

4. Automatisiertes System nach Anspruch 1 oder 2, bei dem die Verteilungsvorrichtung eine von dem Zeitprogrammgeber gesteuerte Fördereinrichtung ist.

5. Automatisiertes System nach einem der Ansprüche 1 bis 4, das unabhängig steuerbare Einspeisequellen für Bakterien und Nährstoffe aufweist.

6. Automatisiertes System nach einem der Ansprüche 1 bis 5, bei dem die Wasserzuführung gefiltert und druckreguliert ist.

7. Automatisiertes System nach einem der Ansprüche 1 bis 6, bei dem die druckbeaufschlagte Luftzuführung eine Aquarien-Luftpumpe ist.

8. Automatisiertes System nach einem der Ansprüche 1 bis 7, bei dem das Öffnungselement (90) eine horizontal angeordnete Drosselblende (98) aufweist, die vertikal in einem kleinen Abstand über der Raum-Auslaßöffnung (92) getrennt angebracht ist.

9. Automatisiertes System nach Anspruch 8, bei dem die Drosselblende (98) einen Außendurchmesser hat, der größer als der Durchmesser der Raum-Auslaßöffnung (92) ist, und eine einzige Öffnung (102) mit einem Durchmesser, der kleiner als der Durchmesser der Raum-Auslaßöffnung (92) ist, aufweist.

10. Automatisiertes System nach einem der Ansprüche 1 bis 7, bei dem das Öffnungselement eine zylindrische Konstruktion mit einem Durchmesser, der näherungsweise gleich dem Durchmesser der Raum-Auslaßöffnung ist, ist und eine Mehrzahl von an seinem Außenumfang angeordneten Öffnungen aufweist, die für eine Fluidverbindung zwischen dem Inneren des Raums und der Raum-Auslaßöffnung sorgen.

11. Automatisiertes System nach einem der Ansprüche 1 bis 10, bei dem die Rückführpumpe eine Zentrifugalpumpe ist.

12. Automatisiertes System nach einem der Ansprüche 1 bis 10, bei dem die Rückführpumpe eine Diaphragmapumpe ist.

## Revendications

1. Système automatisé (10) utile pour cultiver des bactéries à partir d'une population de départ jusqu'à une population d'utilisation dans un intervalle prédéterminé et distribuer ensuite les bactéries pour effectuer une utilisation souhaitée, le système comprenant :
- une chambre de culture biologique (12) ayant une paroi latérale cylindrique avec une surface interne, une partie supérieure et un fond conique ; la partie supérieure comprenant de plus les orifices d'alimentation d'entrée, de l'eau et de l'air et un orifice de décharge ; un orifice de sortie de chambre disposé centralement (22, 92) dans le fond conique ; un élément d'orifice (34, 90) disposé à l'intérieur du fond conique à proximité de l'orifice de sortie ; et un orifice (42) d'entrée du fluide recyclé dans la paroi latérale au niveau d'un point au-dessus du fond conique, l'orifice d'entrée de fluide recyclé (42) étant dirigé tangentiellement le long de la surface interne de la paroi latérale ;
- au moins un minuteur (68) ;
- au moins une source (24) d'alimentation en bactéries et en nutriments communiquant avec l'orifice d'entrée de l'alimentation, ladite source étant contrôlable par ledit minuteur ;
- une source d'eau (80, 50, 82) communiquant avec l'orifice d'entrée de l'eau via une valve qui est contrôlable par ledit minuteur ;
- une alimentation en air pressurisé (60) communiquant avec l'orifice d'entrée de l'air, l'alimentation en air pressurisé étant contrôlable par ledit minuteur ;
- une canalisation de décharge (31) communiquant avec l'orifice de décharge ;
- une pompe de recirculation (62) ayant une entrée communiquant avec l'orifice de sortie (22) de la chambre et une sortie communiquant avec l'orifice (42) d'entrée du fluide recyclé ; et
- une valve (64) disposée entre la pompe de recirculation et l'orifice d'entrée du fluide recyclé (42) qui est contrôlable par ledit minuteur pour dériver le débit de la pompe de recirculation vers une conduite de décharge qui communique aussi sélectivement avec l'orifice (22, 92) de sortie de la chambre via la valve et la pompe de recirculation.

2. Système automatisé selon la revendication 1, dans lequel la source d'alimentation en bactéries et en nutriments communique avec l'entrée d'alimentation via un appareil distributeur.

3. Système automatisé selon la revendication 1 ou 2, dans lequel l'appareil distributeur est une valve contrôlée par le minuteur.

4. Système automatisé selon la revendication 1 ou 2, dans lequel l'appareil distributeur est un convoyeur contrôlé par le minuteur.

5. Système automatisé selon l'une quelconque des revendications 1 à 4, comprenant des sources d'alimentation en nutriments et en bactéries contrôlables indépendamment.

6. Système automatisé selon l'une quelconque des revendications 1 à 5, dans lequel l'alimentation en eau est filtrée et la pression régulée.

7. Système automatisé selon l'une quelconque des revendications 1 à 6, dans lequel l'alimentation en air pressurisé est une pompe à air pour aquarium.

8. Système automatisé selon l'une quelconque des revendications 1 à 7, dans lequel l'élément d'orifice (90) comprend une plaque d'orifice (98) disposée horizontalement, séparée verticalement à une faible distance au-dessus de l'orifice (92) de sortie de la chambre.

9. Système automatisé selon la revendication 8, dans lequel la plaque d'orifice (98) a un diamètre externe plus grand que le diamètre de l'orifice de sortie de la chambre (92) et comprend une ouverture simple (102) ayant un diamètre plus petit que le diamètre de l'orifice de sortie de la chambre (92).

10. Système automatisé selon l'une quelconque des revendications 1 à 7, dans lequel l'élément d'orifice est une structure cylindrique ayant un diamètre approximativement égal au diamètre de l'orifice de sortie de la chambre et comprend une pluralité d'orifices disposés à sa périphérie qui permettent la communication des fluides entre l'intérieur de la chambre et l'orifice de sortie de la chambre.

11. Système automatisé selon l'une quelconque des revendications 1 à 10, dans lequel la pompe de recirculation est une pompe centrifuge.

12. Système automatisé selon l'une quelconque des revendications 1 à 10, dans lequel la pompe de recirculation est une pompe à membrane ou à diaphragme.
